(19) Europäisches Patentamt
European Patent Office
Office européen des brevets

(11) Publication number: **0 211 183**
**A2**

(12) # EUROPEAN PATENT APPLICATION

(21) Application number: 86107881.4

(22) Date of filing: 10.06.86

(51) Int.Cl.[4]: **A 61 K 31/12**

(30) Priority: 11.06.85 JP 126430/85

(43) Date of publication of application: 25.02.87 Bulletin 87/9

(84) Designated Contracting States: BE CH DE FR GB IT LI NL SE

(71) Applicant: Eisai Co., Ltd.
6-10, Koishikawa 4-chome Bunkyo-ku
Tokyo 112(JP)

(72) Inventor: Takasu, Emiko
c/1 Shiseido Co. Ltd. 5-5, Ginza 7-chome
Chuo-ku Tokyo(JP)

(72) Inventor: Koshiba, Suzuko
c/1 Shiseido Co. Ltd. 5-5, Ginza 7-chome
Chuo-ku Tokyo(JP)

(72) Inventor: Morikawa, Fujio
c/1 Shiseido Co. Ltd. 5-5, Ginza 7-chome
Chuo-ku Tokyo(JP)

(72) Inventor: Fujinuma, Yoshimori c/o Shiseido Co. Ltd.
Research Lab. 1050, Niiha-cho Kohoku-ku
Yokohama-shi Kanagawa Prefecture(JP)

(72) Inventor: Furuse, Kazumaro
3-5, Inogashira 2-chome
Mitaka-shi Tokyo(JP)

(74) Representative: Eitle, Werner, Dipl.-Ing. et al,
Hoffmann, Eitle & Partner Patentanwälte Arabellastrasse 4
D-8000 München 81(DE)

(54) **Use of ubidecarenone(ubiquinone-10) as an antichromatotic agent.**

(57) An anti-chromatotic agent is provided, containing a ubiquinone-10 of a formula:

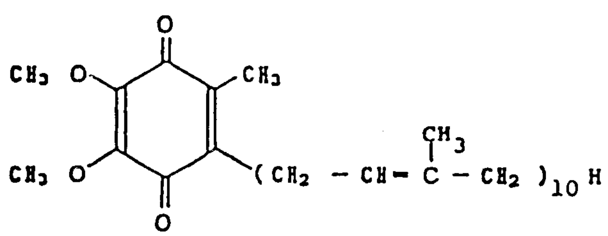

The present agent has an excellent anti-chromatotic effect especially against chromatosis or pigmentation after inflammation. This is safe and less toxic, and is useful as a preventive or remedy for chromatosis or pigmentation, which may continuously be used for a long period of time with high safety. The use of ubiquinone-10 in the preparation of an anti-chromatotic agent is also described.

EP 0 211 183 A2

Croydon Printing Company Ltd.

TITLE MODIFIED
see front page



# Anti-Chromatotic Agents and use thereof

## Field of the Invention

The present invention relates to anti-chromatotic agents, and more precisely, to those of high safety which comprise ubiquinone-10 as an active ingredient, and the use thereof in the preparation of an antichromatotic medicament.

## BACKGROUND OF THE INVENTION

Chromatoses include freckle, chloasma, melasma, senile spot, floriform chromatosis, solar keratosis, nevus pigmentosus, Ohta's nevus, berloque dermatitis, etc. The mechanism of the occurrence of these chromatoses has been unclear in many points up to the present, and in general, it is considered that the chromatoses will result from the hyperpigmentation in a skin due to genetic factors, hormone anomaly, stimulus with ultraviolet ray of sun-light, stimulus to skin with photodynamic substances, aggravation of allergic contact-type dermatitis, etc.

For the remedy of these chromatoses, some means have generally been practised here in Japan, in which a substance capable of inhibiting the formation of melamine, such as vitamin C, vitamin E or glutathione, is internally used by mouth, or vitamin C, cysteine or the like is externally applied to skin. In the West, hydroquinone-containing preparations have been used as medicines.

In addition, various materials have been known, including extracts of crude drugs such as udo (Aralia cordata), cnidium (Cnidium

officinale Makino), aloe, pollen, gardenia (Cape jasmine), soybean, arrowroot (Pueraria thumbergiana), cucurbitaceous plants (Cucurbitaceae), etc. (Japanese Patent Application Laid-Open No. 92208/81, No. 135236/75, No. 77610/82, etc.); pantethine, pantetheine and derivatives thereof (Japanese Patent Application Laid-Open No. 73012/81, No. 74052/82, No. 134022/83, No. 36606/84); vitamin C-related substances such as 3-O-glucosyl-L-ascorbic acid, N-acylamino acid ester-ascorbic acid, 3-O-monoester-ascorbic acid, hydroxypropiophenol-ascorbic acid (Japanese Patent Application Laid-Open No. 27825/84, No. 135411/81, No. 161314/81, No. 101138/76); kojic acid-related substances (Japanese Patent Application Laid-Open No. 3538/78, No. 6432/78, No. 92632/79, No. 7710/81, No. 79616/81, No. 33207/84); hydroquinone-fatty acid esters (Japanese Patent Application Laid-Open No. 145803/82, No. 154507/83); dicarboxylic acid esters (Japanese Patent Application Laid-Open No. 103319/83), etc.

However, ascorbic acids have some problems in the point of the stability, and these are unstable in a moisture-containing system and are often discolored or become to smell disgusting. Thiol compounds such as glutathione or cysteine smell extremely disgusting and are easily oxidized, and these are evaded to be incorporated in preparations for external application. In addition, the production of the effect of these compounds is extremely slow except hydroquinone, and therefore the achievement of the effect is not sufficient.

On the other hand, although the effect of hydroquinone is recognized, this is too stimulative for the skins of Japanese persons,

as having a sensitizing activity, with the result of the occurrence of allergic contact-type dermatitis or the like side-effect, and therefore, the use of the hydroquinone is limitative here in Japan. In addition, this is unstable, when made into preparations. Under the circumstances, a trial for the formation of monoesters with higher fatty acids has heretofore been made, in order to improve the safety of the hydroquinone. However, the esters are to be decomposed with hydrolases in a living body, and therefore, it can hardly be said that these are always safe.

## SUMMARY OF THE INVENTION

In view of the above-described situations in this technical field, the present inventors have earnestly studied various matters in order to obtain such medicines as being highly safe and surely having an excellent anti-chromatotic effect, and as the result thereof, have found that a coenzyme ubiquinone-10, which exists in mitochondrions in organisms and which is considered to participate in an electron-transfer system, is extremely excellent in the safety thereof and may sufficiently achieve an anti-chromatotic effect, and thus have attained the present invention on the basis of this discovery.

Accordingly, the subject matter of the present invention resides in an anti-chromatotic agent comprising ubiquinone-10, which is represented by the following formula, as an active ingredient.

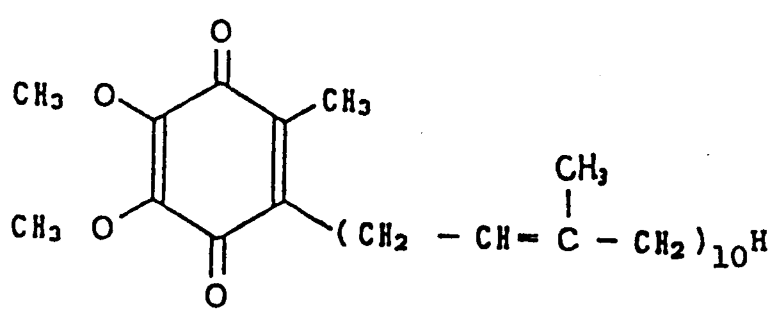

## DETAILED DESCRIPTION OF THE INVENTION

The present invention will be explained in detail hereunder.

The ubiquinone-10 to be used in the present invention is characterized by the number (n) of the isoprenoid side chain of 10. There are some other ubiquinone-n's than this, in accordance with the number of "n". The ubiquinone-10 (where n = 10) among them has the most excellent anti-chromatotic activity for human skins and the safety thereof is highest. The ubiquinone-10 has a melting point of about 48°C and is an yellow to orange-yellow crystalline powder, and this is free from odor or taste. The "chromatosis" as used herein means a pathological pigmentation in a living body or the like symptoms, for example, including freckle, chloasma, melasma, senile spot, floriform chromatosis, solar keratosis, nevus pigmentosus, Ohta's nevus, berloque dermatitis, etc. The ubiquinone-10 is, in particular, extremely effective for chromatoses after inflammation.

(Toxicologic characteristic):

Next, the toxicologic characteristic of the present substance will be explained hereunder.

(1) Acute toxicity:

The acute toxicity of the present substance was investigated in various dosage pathways by the use of Wister rats and ICR-JCL mice. For the peroral or endermic dosage, the present substance was dissolved in a distilled water and the resulting solution was used. For the other dosages, the present substance was dissolved in a physiological salt solution and the resulting solution was dosed with a stomach conductor or an injector. In each dosage, a determined amount of the substance was regulated and properly dosed.

After the dosage, the expression of toxicopathy was continuously observed, and the lethality with the lapse of time for 7 days was measured and the value of $LD_{50}$ was obtained therefrom. Both the tested animals as living and the tested animals as died were dissected for observation. $LD_{50}$ was calculated on the basis of Litchfield-Wilcoxon figure-calculation method.

The results from the above tests are given in the following Table-1, which prove that the present substance has a high $LD_{50}$ value in every dosage and therefore is safe as a medicine.

**Table-1**

| | $LD_{50}$ value (95% confidence limit) | |
|---|---|---|
| Dosage pathway | rats(male, female) | mice (male, female) |
| Peroral | 4000mg/kg | 4000mg/kg |
| Endermic | 4000 | 4000 |
| Hypodermic | 500 | 500 |
| Intramuscular | 500 | 500 |
| Intra-abdominal | 1500 | 1500 |
| Intravenous | 250 | 250 |

In the above-described dosage, no animals as tested died. In addition, no abnormal change was found in the tested animals on the point of the general condition, the weight, the amount of feed intake and the observation in dissection.

(2) Sensitization:

This was tested in accordance with a maximization test by Magnusson B. and Kligman, A.M. The test is as follows:

(i) Induction:

Twenty guinea pigs were prepared for the test, ten being for the induction and the other ten being for the control in the challenge. The suprascapular part of the guinea pig was clipped and shaved in the area of 4cm x 6cm, and the following three pairs of hypodermic injections (a), (b) and (c) were imparted simultaneously to both the right and left parts as being symmetrical about the median line.

(a) Each 0.05ml of FCA to two points in both the right and left parts, totalling 0.1ml in all.

(b) Each 0.05ml of the test substance to two points in both the right and left parts, in the form of an aqueous solution (content of test substance: 10%).

(c) Each 0.05ml of FCA as containing the test substance emulsion to two points in both the right and left parts.

After one week from the hypodermic injection, the suprascapular part was again clipped and vaseline containing 10% of sodium laurylsulfate was applied thereover thereby to cause a slight inflammation on the applied part. By this treatment, the sensitization was intensified. Next, the test substance was applied to this part, which was thereafter airtightly sealed.

(ii) Challange:

In the 21th day from the beginning of the induction treatment, the lateral region of the abdomen of each guinea pig of the induction

group and the control group was clipped and shaved in the area of 5cm x 5cm, and the test substance as diluted with a pertinent solvent was applied thereto and the applied part was airtightly sealed for 24 hours. The control group was treated in the same manner.

(iii) Evaluation:

| | | |
|---|---|---|
| i. | No change with the naked eye: | 0 |
| ii. | Erythema of light or slight degree: | 1 |
| iii. | Erythema of middle degree: | 2 |
| iv. | Erythema and edema of heavy degree: | 3 |

(iv) Result:

No sensitization reaction was admitted in all guinea pigs as tested. This means that the present substance is safe in the continuous endermic use thereof for a long period of time.

(Pharmaceutical effect):

(1) Anti-chromatotic effect and side-effect:

Using Weiser Maple GP with phototoxic pigmentation by 8MOP treatment, 50μl of a test sample was applied to the back region thereof as shaved in the range of about $4cm^2$, once a day for eight weeks. The anti-chromatotic effect and the degree of the side-effect pigmentation as appeared were measured by means of four-point evaluation. (+) designates discoloration effect; and (-) designates side-effect. Samples as used herein were 5%-hydroquinone (as dissolved in PG) and 5%-ubiquinone-10 (as dissolved in acetone).

(i) Evaluation: (Discoloration effect and pigmentation)

Table-2

| Total Determination | Evaluation point | Visual Determination |
|---|---|---|
| Anti-chromatotic effect: | | |
| + | 3 | Whitened |
| ± | 2 | Somewhat whitened |
| - ∿ ± | 1 | Slightly whitened |
| - | 0 | No change |
| Side-effect of pigmentation: | | |
| - | 0 | No change |
| - ∿ ± | -1 | Somewhat blackened |
| ± | -2 | Blackened |
| + | -3 | Apparently blackened |

Table-3 (Result)

| Applied medicine | Period of application (week) 1 | 2 | 3 | 4 | 5 | 6 | 7 | 8 |
|---|---|---|---|---|---|---|---|---|
| HQ | 0.4 | 0.7 | 1.2 | 0.8 | 0.3 | 0.2 | -0.5 | -0.8 |
| Present substance | 0.6 | 1.5 | 1.8 | 1.1 | 0.8 | 0.2 | 0 | 0 |

(Manufacture of preparations):

Next, the means of how to manufacture the preparations of the anti-chromatotic agent containing the present ubiquinone will be explained below.

For the manufacture of the anti-chromatotic agent of the present invention, a mixture of ubiquinone and pharmaceutically acceptable base

materials optionally together with some other phamaceutical substances is used. In particular, the co-use of ubiquinone and an ultraviolet-absorbent for the purpose of shielding of ultraviolet ray is more effective for the prevention and the remedy of chromatosis.

Examples of usable base materials are vehicles such as lactose, starch, calcium carbonate, magnesium meta-silicate-aluminate, magnesium aluminium hydroxide, calcium hydrogen-phosphate, sugar, lactose, aluminium silicate and fine crystalline cellulose; binders such as carboxymethyl cellulose, polyvinyl pyrrolidone, gum arabic, gelatin, sodium alginate and hydroxypropyl starch; lubricants such as talc, stearic acid, calcium stearate, magnesium stearate and zinc stearate; moisture-retentives such as glycerin, propyleneglycol and sorbitol; disintegrators such as agar, silicic anhydride and calcium carboxymethyl-cellulose; and other surfactants, buffers, preservatives, perfumes, dyes, oils, pigments, water, alcohols, tackifiers, antiseptics, antioxidants and chelating agents. These may be used singly or in the form of a mixture of two or more of them. These examples are not whatsoever limitative in the present invention.

Examples of usable pharmaceutical substances are skin nutrients, hair tonics, enzymes and conventional whitening agents.

For instance, iodotyrosine and derivatives thereof, amino acids such as methionine, lysine and serine and derivatives thereof, vitamins such as vitamin A, pantothenic acid, biotin, vitamin $B_2$, vitamin $B_6$, nicotinic acid, vitamin C, vitamin E, vitamin F and derivatives thereof, enzymes and hormones such as female sex hormone, pituitary hormone

and phosphorylase, and other inositol, orotic acid, thioctic acid, chondroitin sulfuric acid and hyaluronic acid may be used, which however, are not whatsoever limitative.

Examples of usable ultraviolet absorbents are benzoic acid-type UV-absorbents such as para-amino-benzoic acid (hereinafter referred to as PABA in short), PABA-monoglycerin-diester, N,N-dipropoxy-PABA-ethylester, N,N-diethoxy-PABA-ethylester, N,N-dimethyl-PABA-ethylester, N,N-dimethyl-PABA-butylester, N,N-dimethyl-PABA-amylester, N,N-dimethyl-PABA-octylester, etc.; anthranilic acid-type UV-absorbents such as homomenthyl N-acetyl-anthranilate, etc.;salicylic acid-type UV-absorbents such as amyl salicylate, menthyl salicylate, homomenthyl salicylate, octyl salicylate, phenyl salicylate, benzyl salicylate, p-isopropanol-phenyl salicylate, etc.; cinnamic acid-type UV-absorbents such as octyl cinnamate, ethyl 4-isopropyl-cinnamate, methyl 2,5-diisopropyl-cinnamate, ethyl 2,4-diisopropyl-cinnamate, methyl 2,4-diisopropyl-cinnamate, propyl p-methoxy-cinnamate, isopropyl p-methoxy-cinnamate, isoamyl p-methoxy-cinnamate, octyl methoxy-cinnamate, 2-ethoxyethyl p-cinnamate, cyclohexyl p-methoxy-cinnamate, ethyl α-cyano-β-phenyl-cinnamate, 2-ethylhexyl α-cyano-β-phenyl-cinnamate, glyceryl mono-2-ethylhexanoyl-diparamethoxy-cinnamate, etc.; benzophenone-type UV-absorbents such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenylbenzophenone-2-carboxylate, 2-hydroxy-4-n-octoxy-

benzophenone, 4-hydroxy-3-carboxybenzophenone, etc.; and other UV-absorbents such as 3-(4'-methylbenzylidene)-d,l-camphor, 3-benzylidene-d,l-camphor, urocanic acid, ethyl urocanate, 2-phenyl-5-methylbenzoxazole, 2,2'-hydroxy-5-methylphenylbenzotriazole, 2-(2'-hydroxy-5'-t-octyl-phenyl)-benzotriazole, 2-(2'-hydroxy-5'-methyl-phenyl)benzotriazole, dibenzaladine, dianisoylmethane, 4-methoxy-4'-t-butyl-dibenzoylmethane, 5-(3,3'-dimethyl-2-norbornylidene)-3-pentan-2-one, benzal-phthalide, etc. However, these are not limitative.

For the form of the preparations of the present anti-chromatotic agent, any forms which are generally used for conventional medicines, non-medicines and cosmetics may be used, so far as they are suitable for attaining the pharmaceutical effect of the anti-chromatotic agent, for example, including liquids for external application such as lotion, liniment, aqueous solution and emulsion; solids for external application such as powder and soluble tablets; semi-solids for external application such as cream, pack, ointment, paste and jelly; and soaps. In addition, internal medicines or injections may be used, as being effective in some cases.

The content of the active ingredient of ubiquinone in the anti-chromatotic agent of the present invention is 0.001-20% by weight, preferably 0.2-10% by weight, especially preferably 1-5% by weight. Sufficient effect may be attained when up to 20% by weight of the active ingredient is incorporated in the present agent, and if a more amount thereof is incorporated, no more intensification of the effect can often be achieved.

(Means for dosage or application and amount of dose):

The main use of the anti-chromatotic agent of the present invention is an endermic or external application to directly apply the agent to the affected part, and in addition, the present agent may be given to a patient by means of peroral administration, hypodermic application, intravenous injection, intramuscular injection and intra-abdominal injection.

The amount of the dose of the anti-chromatotic agent of the present invention often varies, depending upon the age, the individual difference, the condition of the disease or the like factors in the patients. In general, 0.005-10mg/$cm^2$ or so of ubiquinone-10 is coated or sprayed over the affected part several times a day, in case of an external application, which however, is not whatsoever limitative. As the case may be, the amount falling without the said range may be used case by case or in accordance with the object of the use of the agent.

(Effect):

In order to clarify the anti-chromatotic effect of ubiquinone-10 which is used in the present invention, the following experiment was actually carried out, using a ubiquinone-10-containing pack.

Test Method: 160 panelers to be tested, who were affected by chromatosis in their faces, were prepared for the test. To 80 panelers among them was applied the agent of the Example 1 (as given hereinafter) twice or three times a week, by coating the agent to their faces, for continuous three months. To the remaining 80 panelers was applied a comparative agent, which was same as the agent of the Example 1 with the exception that

the ubiquinone-10 in the Example 1 was replaced by water, in the same manner as above. After the continuous application for 3 months, the degree of the discoloration of the faces in both groups was measured by a doctor with the naked eye and the effect of the agents as used was evaluated therefrom.

Effect: The effect was as follows:

Table-4

| Kind of Chromatosis | General observation | | | | |
|---|---|---|---|---|---|
| | Fairly cured | Somewhat cured | No change | Aggreviated | Total of persons tested |
| Chloasma, Freckle | 10 | 14 | 6 | 0 | 30 |
| Senile spot | 5 | 8 | 2 | 0 | 15 |
| Pigmentation after inflammation | 20 | 3 | 0 | 0 | 23 |
| Others | 2 | 4 | 6 | 0 | 12 |
| Total | 37 persons | 29 persons | 14 persons | 0 person | 80 persons |
| | 46.3% | 36.3% | 17.5% | 0% | 100% |
| | 82.6% (Fairly cured + Somewhat cured) | | | | |

The above results apparently prove that the anti-chromatotic pack of the present invention, containing 0.5% by weight of ubiquinone-10, is highly effective for cure of chromatosis and that the effectiveness factor thereof is 82.6%.

In particular, the present agent is especially effective against

pigmentation after inflammation. The present agent is safe and less toxic and therefore may be used as a preventive or remedy for chromatosis, and the continuous use of the agent for a long period of time is possible with high safety. In addition, the present agent is free from any side-effect, and therefore may effectively be used for prevention of sunburn and acceleration of healing thereof.

The present invention will be explained in greater detail by reference to the following examples, which, however, are not intended to be interpreted as limiting the scope of the present invention. All amounts are % by weight, unless otherwise specifically indicated.

Example 1:

| | |
|---|---|
| (Alcoholic phase) | % by weight |
| 95%-Ethanol | 10.0 |
| Polyoxyethylene(15 moles)-oleylalcohol-ether | 2.0 |
| Olive oil | 2.0 |
| Ubiquinone-10 | 0.5 |
| Antiseptic | appropriate amount |
| Perfume | appropriate amount |
| (Aqueous phase) | |
| Polyvinyl alcohol | 12.0 |
| Glycerin | 3.0 |
| Polyethylene glycol 1500 | 1.0 |
| Ion-exchanged water | balance |

Manufacture:

The aqueous phase was prepared at 80°C and cooled to 50°C. The alcohol phase except olive oil and ubiquinone-10 was prepared at room temperature and was added to the aqueous phase, and thereafter the ubiquinone-10 as dissolved in the olive oil was added thereto and uniformly blended and then, the whole was kept cooled.

Example 2:

| | % by weight |
|---|---|
| Stearic acid | 6.0 |
| Sorbitan monostearate | 2.0 |
| Polyoxyethylene(20 moles)-sorbitan monostearate | 1.5 |
| Propylene glycol | 10.0 |
| Ubiquinone-10 | 0.2 |
| Antiseptic, Antioxidant | appropriate amount |
| Perfume | appropriate amount |
| Ion-exchanged water | balance |

Manufacture:

The propylene glycol was added to the ion-exchanged water and heated at 70°C (aqueous phase). The other components were blended and molten under heat and kept at 70°C (oily phase). The oily phase was added to the aqueous phase and pre-emulsified and then uniformly emulsified in a homo-mixer. Afterwards, the whole was cooled to 30°C, while well stirred.

Example 3:

| | % by weight |
|---|---|
| Stearyl alcohol | 7.0 |
| Stearic acid | 2.0 |
| Hydrous lanolin | 2.0 |
| Squalane | 5.0 |
| 2-Octyldodecyl alcohol | 6.0 |
| Polyoxyethylene(25 moles)-cetylalcohol-ether | 3.0 |
| Glycerin monostearate | 2.0 |
| Ubiquinone-10 | 10.0 |
| Propylene glycol | 5.0 |
| Antiseptic, Antioxidant | appropriate amount |
| Perfume | appropriate amount |
| Ion-exchanged water | balance |

Manufacture:

The propylene glycol was added to the ion-exchanged water and heated and kept at 70°C (aqueous phase). The other components were blended and molten under heat and kept at 70°C (oily phase). The oily phase was added to the aqueous phase and pre-emulsified and then uniformly emulsified in a homo-mixer. Afterwards, the whole was cooled to 30°C, while well stirred.

Example 4:

| | % by weight |
|---|---|
| Solid parafin | 5.0 |
| Bees wax | 10.0 |

| | |
|---|---|
| Vaseline | 15.0 |
| Liquid paraffin | 41.0 |
| Glycerin monostearate | 2.0 |
| Polyoxyethylene(20 moles)-sorbitan monolaurate | 2.0 |
| Soap powder | 0.1 |
| Ubiquinone-10 | 10.0 |
| Borax | 0.2 |
| Antiseptic, Antioxidant | appropriate amount |
| Perfume | appropriate amount |
| Ion-exchanged water | balance |

Manufacture:

The soap powder and the borax were added to the ion-exchanged water and dissolved under heat and kept at 70°C (aqueous phase). The other components were blended and molten under heat and kept at 70°C (oily phase). The oily phase was gradually added to the aqueous phase, while stirred, and reacted. After the reaction, the whole was uniformly emulsified in a homo-mixer, and after the emulsification, the emulsion was cooled to 30°C, while well stirred.

Example 5:

| | % by weight |
|---|---|
| Stearic acid | 2.5 |
| Cetyl alcohol | 2.0 |
| Vaseline | 5.0 |
| Liquid paraffin | 10.0 |

| | |
|---|---|
| Polyoxyethylene(10 moles)-mono-oleate | 2.0 |
| Polyethylene glycol-1500 | 1.0 |
| Triethanolamine | 1.0 |
| Ubiquinone-10 | 5.0 |
| Antiseptic, Antioxidant | appropriate amount |
| Perfume | appropriate amount |
| Ion-exchanged water | balance |

Manufacture:

The polyethylene glycol-1500 and the triethanolamine were added to the ion-exchanged water and dissolved under heat and kept at 70°C (aqueous phase). The other components were blended and molten under heat and kept at 70°C (oily phase). The oily phase was added to the aqueous phase and pre-emulsified and then uniformly emulsified in a homo-mixer. After the emulsification, the whole was cooled to 30°C, while well stirred.

| Example 6: | % by weight |
|---|---|
| Stearic acid | 1.5 |
| Cetyl alcohol | 0.5 |
| Bees wax | 2.0 |
| Polyoxyethylene(20 moles)-mono-oleate | 1.0 |
| Glycerin mono-stearate | 1.0 |
| Ubiquinone-10 | 3.0 |
| Quince seed-extract (5%-aqueous solution) | 20.0 |
| Propylene glycol | 5.0 |

| | |
|---|---|
| Ethyl alcohol | 10.0 |
| Antiseptic, Antioxidant | appropriate amount |
| Perfume | appropriate amount |
| Ion-exchanged water | balance |

Manufacture:

The propylene glycol was added to the ion-exchanged water and dissolved under heat and kept at 70°C (aqueous phase). The perfume and ultraviolet-absorbent were added to the ethyl alcohol and dissolved (alcoholic phase). The other components except quince seed-extract were blended and dissolved under heat and kept at 70°C (oily phase). The oily phase was added to the aqueous phase and pre-emulsified and then, the whole was uniformly emulsified in a homo-mixer. The alcoholic phase and the quince seed-extract were added thereto, while stirred. Afterwards, the whole was cooled to 30°C, while well stirred.

Example 7:

| | % by weight |
|---|---|
| Micro-crystalline wax | 1.0 |
| Bees wax | 2.0 |
| Lanolin | 2.0 |
| Liquid paraffin | 20.0 |
| Squalane | 10.0 |
| Sorbitan sesqui-oleate | 4.0 |
| Polyoxyethylene(20 moles)-sorbitan mono-oleate | 4.0 |
| Ubiquinone-10 | 2.0 |

| | |
|---|---|
| Antiseptic, Antioxidant | appropriate amount |
| Perfume | appropriate amount |
| Ion-exchanged water | balance |

Manufacture:

The propylene glycol was added to the ion-exchanged water and dissolved under heat and kept at 70°C (aqueous phase). The other components were blended and molten under heat and kept at 70°C (oily phase). The aqueous phase was gradually added to the oily phase, while stirred, and then uniformly emulsified in a homo-mixer. After the emulsification, the whole was cooled to 30°C, while well stirred.

Example 8:

| | % by weight |
|---|---|
| (Alcoholic phase) | |
| 95%-ethanol | 25.0 |
| Polyoxyethylene(40 moles)-hardened castor oil-ether | 4.0 |
| Ubiquinone-10 | 20.0 |
| Antiseptic, antioxidant | appropriate amount |
| Perfume | appropriate amount |
| (Aqueous phase) | |
| Dipropylene glycol | 15.0 |
| Glycerin | 5.0 |
| Sodium hexameta-phosphate | appropriate amount |
| Ultraviolet absorbent | appropriate amount |
| Ion-exchanged water | balance |

Manufacture:

The aqueous phase and the alcoholic phase were prepared and then dissolved.

Example 9:

| | % by weight |
|---|---|
| 96%-ethanol | 10.0 |
| Dipropylene glycol | 15.0 |
| Ubiquinone-10 | 5.0 |
| Polyoxyethylene(15 moles)-oleyl alcohol | 5.0 |
| Carboxyvinyl polymer (trade name: Carbopol 941) | 1.0 |
| Caustic soda | 0.15 |
| L-arginine | 0.1 |
| Ultraviolet-absorbent | appropriate amount |
| Antiseptic, Antioxidant | appropriate amount |
| Perfume | appropriate amount |
| Ion-exchanged water | balance |

Manufacture:

The Carbopol 941 was uniformly dissolved in the ion-exchanged water. On the other hand, the dipropylene glycol-ether and the other components were dissolved in the 95%-ethanol and the resulting solution was added to the aqueous phase. Next, this was neutralized and tackified with the caustic soda and the L-arginine.

Example 10:

| | % by weight |
|---|---|
| Stearic acid | 5.0 |
| Stearyl alcohol | 4.0 |
| Butyl stearate | 8.0 |
| Glycerin monostearate | 2.0 |
| Ubiquinone-10 | 1.0 |
| Propylene glycol | 20.0 |
| Caustic potash | 0.2 |
| Antiseptic, Antioxidant | appropriate amount |
| Perfume | appropriate amount |
| Ion-exchanged water | balance |

Manufacture:

The propylene glycol and the caustic potash were added to the ion-exchanged water and dissolved and then kept at 70°C under heat (aqueous phase). The other components were blended and dissolved under heat and kept at 70°C (oily phase). The oily phase was gradually added to the aqueous phase, and after the complete addition, the whole was kept at the said temperature for a while for the reaction of the components. Afterwards, the whole was uniformly emulsified in a homo-mixer and then cooled to 30°C, while well stirred.

Example 11:

| | % by weight |
|---|---|
| (Alcoholic phase) | |
| 95%-ethanol | 2.0 |

| | |
|---|---|
| Antiseptic | appropriate amount |
| Perfume | appropriate amount |
| Dye | appropriate amount |
| Olive oil | 2.0 |
| Ubiquinone-10 | 0.001 |
| (Aqueous phase) | |
| Propylene glycol | 7.0 |
| Zinc flower | 25.0 |
| Kaolin | 20.0 |
| Ion-exchanged water | balance |

Manufacture:

The aqueous phase was uniformly prepared at room temperature. Next, the alcoholic phase except the olive oil and the ubiquinone-10 was prepared at room temperature and then added to the aqueous phase. Afterwards, the ubiquinone-10 as dissolved in the olive oil was added thereto and uniformly blended.

(Effect of the Invention):

The ubiquinone-10 has an excellent anti-chromatotic effect, and in particular, is effective against chromatosis or pigmentation after inflammation, as apparent from the above-described experiments. In addition, this is safe and less toxic, and therefore useful as a preventive or remedy for chromatosis or pigmentation, which may continuously be used for a long period of time with high safety. Further, this is free from any side-effect, and therefore may effectively be used for prevention of sunburn and acceleration of healing thereof.

WHAT IS CLAIMED IS:

1. An anti-chromatotic agent containing a ubiquinone-10 of a formula:

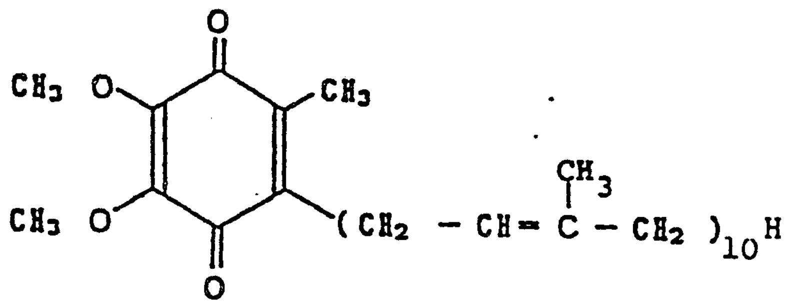

2. The anti-chromatotic agent as claimed in claim 1, wherein the concentration of the ubiquinone-10 is 0.001-20% by weight.

3. The anti-chromatotic agent as claimed in claim 2, wherein the concentration of the ubiquinone-10 is 0.2-10% by weight.

4. Use of the ubiquinone-10 of claim 1 in the preparation of an anti-chromatotic medicament.